# EUROPEAN PATENT SPECIFICATION

(11) **EP 1 438 018 B1**
(45) Date of publication and mention of the grant of the patent: **11.08.2010**
(21) Application number: 02799685.9
(22) Date of filing: 28.09.2002
(51) Int. Cl.: A61K 9/00, A61K 9/28, A61K 9/20

(54) **FONDANT-BASED PHARMACEUTICAL COMPOSITION**
PHARMAZEUTISCHE ZUSAMMENSETZUNG AUF FONDANTBASIS
COMPOSITION PHARMACEUTIQUE A BASE DE FONDANT

(30) Priority: 28.09.2001 US 966939; 28.09.2001 US 966509; 28.09.2001 US 966497; 28.09.2001 US 967414; 28.09.2001 US 966450
(43) Date of publication of application: 21.07.2004
(73) Proprietor: McNeil-PPC, Inc., Skillman, New Jersey 08558 (US)
(72) Inventor: SOWDEN, Harry, S., Glenside, PA 19038 (US); BUNICK, Frank, J., Quakertown, PA 18951 (US); LABELLA, Gus, B., Collegeville, PA 19426 (US)
(74) Representative: James, Anthony Christopher W.P.
(86) International application number: PCT/US2002/031067
(87) International publication number: WO 2003/026613

(56) References cited:
- EP-A- 0 455 599
- EP-A- 0 646 650
- EP-A- 0 834 516
- WO-A-02/19833
- US-A- 3 627 583
- US-A- 4 230 693
- US-A- 4 425 332
- US-A- 4 851 226
- US-A- 5 578 336

## Description

### 1. Field of the Invention

The present invention relates to a fondant-based pharmaceutical composition and dosage forms made therefrom.

### 2. Description of the Prior Art

Pharmaceuticals intended for oral administration are typically provided in solid form as tablets, capsules, pills, lozenges, or granules. Tablets are swallowed whole, chewed in the mouth, or dissolved sublingually. Soft tablets that either are chewed or dissolved in the mouth are often employed in the administration of pharmaceuticals where it is impractical to provide a tablet for swallowing whole. Soft tablets are advantageous where it is desirable to make an active ingredient available topically in the mouth or throat for both local effects or systemic absorption. Soft tablets are also utilized to improve drug administration in pediatric and geriatric patients. Soft tablets designed to disintegrate in the mouth prior to swallowing are particularly useful for improving compliance of pediatric patients.

Generally, soft tablets are made by direct compaction of a mixture of tabulating compounds including an active ingredient, flavoring, binders, etc. The mixture is fed into a die cavity of a tablet press and a tablet is formed by applying pressure. Hardness of the resulting tablet is a direct function of the compaction pressure employed and the compactibility of the ingredients in the formulation. A softer tablet, having an easier bite-through, may be prepared by employing reduced compaction pressures. The resulting tablet is softer, but also more fragile, brittle, and easily chipped.

Soft tablets designed to disintegrate in the mouth without chewing are disclosed by Cousin et al., in U.S. Patent No. 5,464,632, and Wehling et al., in U.S. Patent Nos. 5,223,264 and 5,178,878. While these soft tablets for oral administration advantageously disintegrate completely in the mouth prior to swallowing, they have the disadvantage of being highly friable, requiring costly specialized handling and packaging in order to prevent breakage.

It is known to apply outer coatings to a chewable tablet in order to protect the soft core. Typically, such outer coatings contain cellulose derivatives as major ingredients, which have relatively high melting points, i.e., greater than 135° C. For example, PCT Application No. WO 93/13758 discloses the application of a thin layer of coating material such as a disaccharide, polysaccharide, or cellulose derivative onto a compressed tablet. U.S. Patent No. 4,828,845 relates to the coating of a comestible with a coating solution comprising xylitol, a film-forming agent such as methyl cellulose, a binder, optionally a filler, and optionally a plasticizer such as polyethylene glycol, the balance of the solution being water. The plasticizer makes up only about 3 to 7 weight percent of the coating solution disclosed in the '845 patent. U.S. Patent No. 4,327,076 discloses a compressed, soft, chewable tablet containing an antacid or other active ingredient that may be coated with a sealant or a spray coat of chocolate.

Alternatively, as disclosed in U. S. Patent No. 4,684,534, moisture-free soft tablets have been produced by compressing a combination of an active ingredient with a carbohydrate and a binder such that the open pore structure of the combination is destroyed only at the tablet surface. Because of their relatively hard exterior, these tablets are resistant to moisture absorption; however, these tablets quickly liquefy and melt when chewed due to their open pore interior structure.

Food products having soft or liquid centers, layers or other areas are formulated by arranging two fat-containing components contiguous with one another. A fat in the first component migrates into the second, forming a mixture having a lower solids content than the second fat, while the structural integrity of the first component is maintained. The process is especially adapted to the formation of soft- and liquid-centered confections. One preferred embodiment employs fats bearing long, saturated C₁₆ to C₂₂ fatty acid residues and a mixture of short C₂ to C₄ acid residues, preferably containing acetic acid residues, as the migrating fat in a confectionery coating, and hydrogenated coconut or palm kernel oil as the fat in the confectionery center. An especially preferred embodiment employs, as the migrating fat, triglycerides bearing long, saturated substituents containing at least about 75% stearic acid residues and short residues derived from acetic acid, a mixture of acetic and propionic acid, or a mixture of acetic and butyric acid. Since sucrose and invertase are not essential elements of the center, artificial sweeteners can be used to replace all or part of the sucrose, resulting in reduced calorie confections. Caloric reduction is further enhanced because preferred migrating fats are low in calories. Yet another method for preparing soft centers in food products is disclosed in U.S. Patent No. 5,362,508, wherein a center composition comprising a mixture of sucrose, invertase, and a fat component is coated with a second fat component. Upon incubation, short chain fatty acid residues from the second fat component migrated into the center fat component to yield a soft fat mixture in the center having a lower fat solids content.

It has now been discovered that active ingredients such as pharmaceuticals or nutritional products may be added to a novel, quick-melting fondant-based pharmaceutical composition that imparts a silky smooth texture during ingestion. This composition not only effectively masks the taste and texture of the active ingredient, particularly large particle sized active ingredients, but it conveniently may be consumed anywhere without the need for water. The fondant-based pharmaceutical composition may be compressed then coated with one or more outer coatings made of conventional coating materials, such as saccharides, cellulose derivatives, fats and waxes, and the like. Application of a protective coating according to the invention not only stabilizes the friability of the dosage form, but also effectively provides a water-resistant barrier that prevents the dosage form from drying out thereby allowing for the gradual softening of the fondant core.

### Summary of the Invention

The invention provides a fondant-based pharmaceutical composition comprising an active ingredient and a carbohydrate, at least a portion of which carbohydrate is crystallized and has an average particle size of 2 to 35 microns, said composition having a moisture content in the range of 10 to 13 percent, and wherein said active ingredient is in the form of particles having an average particle size of 200 to 1200 microns

The invention also provides a dosage form comprising: a) a fondant-based pharmaceutical composition comprising an active ingredient and a carbohydrate, at least a portion of which carbohydrate is crystallized and has an average particle size of 2 to 35 microns, said composition having a moisture content in the range of 10 to 13 percent and wherein said active ingredient is in the from of particles having an average particle size of 200 to 1200 microns, and b) at least one coating overlying said composition.

Finally, the invention further provides a method for making a soft tablet comprising: (a) forming a tablet containing an active ingredient wherein said active ingredient is in the from of particles having an average particle size of 200 to 1200 microns, and a hydrolyzable carbohydrate to a hardness of 2 to 10 kp/cm2; (b) adding water to the tablet before, during or after step (a); and (c) adding a hydrolase, e.g. a glycosidase to the tablet before, during or after step (a).

### Detailed Description of the Invention

Fondants as known in the confectionery industry are sugar confectionery products that contain mixed sugars held in two phases. Sugar crystals, typically having a particle size in the range of 2 to 35 microns, constitute the solid phase of these products. They are evenly dispersed in a high sugar solids syrup or liquid phase, which is saturated with respect to the crystallized sugars. The liquid phase typically constitutes 35 to 50 % by weight of the fondant.

The present fondant-based pharmaceutical composition comprises one or more active ingredients wherein said active ingredient is in the from of particles having an average particle size of 200 to 1200 microns, and one or more carbohydrates, at least a portion of which carbohydrate(s) are crystallized and have an average particle size of 2 to 35 microns. The composition has a moisture content in the range of 5 to 15 percent, e.g. 10 to 13 percent.

As used herein, the term "dosage form" applies to any solid object, semi-solid, or liquid composition designed to contain a specific pre-determined amount (i.e. dose) of a certain ingredient, for example an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutriceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human.

The term "active ingredient" is used herein in a broad sense and encompasses any material that can be carried by or entrained in a dosage form. Suitable active ingredients for use in this invention include for example pharmaceuticals, minerals, vitamins and other nutriceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, oral contraceptives, polydimethylsiloxanes, respiratory agents, sleep-aids, urinary tract agents and mixtures thereof.

Preferred pharmaceuticals for use as the active ingredient include acetaminophen, ibuprofen, flurbiprofen, ketoprofen, naproxen, diclofenac, aspirin, pseudoephedrine, phenylpropanolamine, chlorpheniramine maleate, dextromethorphan, diphenhydramine, famotidine, loperamide, ranitidine, cimetidine, astemizole, terfenadine, fexofenadine, cetirizine, antacids, mixtures thereof and pharmaceutically acceptable salts thereof. More preferably, the active ingredient is selected from the group consisting of acetaminophen, ibuprofen, pseudoephedrine, dextromethorphan, diphenhydramine, chlorpheniramine, calcium carbonate, magnesium hydroxide, magnesium carbonate, magnesium oxide, aluminum hydroxide, mixtures thereof, and pharmaceutically acceptable salts thereof. Active ingredients may further include but are not limited to food acids; insoluble metal and mineral hydroxides, carbonates, oxides, polycarbophils, and salts thereof; adsorbates of active drugs on a magnesium trisilicate base and on a magnesium aluminum silicate base.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, anti-inflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarboxylic acid derivatives, e.g. diflunisal, flufenisal, and the like; and oxicams, e.g. piroxicam, sudoxicam, isoxicam, meloxicam, and the like. In a particularly preferred embodiment, the active ingredient is selected from propionic acid derivative NSAIDs, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In a particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, doxylamine, astemizole, terfenadine, fexofenadine, loratadine, cetirizine, mixtures thereof and pharmaceutically acceptable salts, esters, isomers, and mixtures thereof.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

Examples of suitable polydimethylsiloxanes, which include, dimethicone and simethicone, are those disclosed in United States Patent Nos. 4,906,478, 5,275,822, and 6,103,260. As used herein, the term "simethicone" refers to the broader class ofpolydimethylsiloxanes, including simethicone and dimethicone.

The active ingredient is dispersed or dissolved in the fondant-based pharmaceutical composition. The active ingredient is present in the form of particles. The average particle size of the active ingredient is relatively large, i.e., 200 to 1200 microns, preferably 250 to 350 microns. It has been found that the fondant-based pharmaceutical composition is particularly useful for masking the texture of large particles of active ingredient.

The active ingredient(s) are present in the fondant-based pharmaceutical composition in a therapeutically effective amount, which is an amount that produces the desired therapeutic response upon oral administration and can be readily determined by one skilled in the art. When determining this amount, the particular compound being administered, the bioavailability characteristics of the active ingredient, the dose regime, the age and weight of the patient, and other factors must be considered. Typically, the active ingredient is present in the fondant-based pharmaceutical composition in an amount of 1 to 50 weight percent, e.g. from 5 to 30 weight percent, or from 2 to 15 weight percent, or from 15 to 40 percent by weight based on the total weight of fondant-based pharmaceutical composition. In embodiments in which the fondant-based pharmaceutical composition is further surrounded by one or more coatings in a pharmaceutical dosage form, the active ingredient is typically present in the dosage form in an amount of 0.5 to 50 weight percent of the dosage form, e.g. 0.5 to 30 weight percent of the dosage form.

Suitable carbohydrates include, but are not limited to crystallizable carbohydrates. Suitable crystallizable carbohydrates include the monosaccharides and the oligosaccharides. Of the monosaccharides, the aldohexoses e.g., the D and L isomers of allose, altrose, glucose, mannose, gulose, idose, galactose, tagatose, talose, and the ketohexoses e.g., the D and L isomers of fructose and sorbose along with their hydrogenated analogs: e.g., glucitol (sorbitol), and mannitol are preferred. Of the oligosaccharides, the 1,2-disaccharides sucrose, trehalose, and turanose, the 1,4-disaccharides maltose, lactose, and cellobiose, and the 1,6-disaccharides gentiobiose and melibiose, as well as the trisaccharide raffinose are preferred along with the isomerized form of sucrose known as isomaltulose and its hydrogenated analog isomalt. Other hydrogenated forms of reducing disaccharides (such as maltose and lactose), for example, maltitol and lactitol are also preferred. Additionally, the hydrogenated forms of the aldopentoses: e.g., D and L ribose, arabinose, xylose, and lyxose and the hydrogenated forms of the aldotetroses: e.g., D and L erythrose and threose are preferred and are exemplified by xylitol and erythritol, respectively. Preferred crystallizable carbohydrates for making the fondant-based pharmaceutical composition of the invention include sugars and polyhedric alcohols. Preferred sugars include sucrose, dextrose, dextrose monohydrate, fructose, maltose, xylose, lactose, and mixtures thereof. Preferred polyhedric alcohols include mannitol, sorbitol, maltitol, xylitol, erythritol, isomalt and mixtures thereof. Sucrose is particularly preferred.

At least a portion of the carbohydrate is crystallized and has an average particle size of 2 to 35 microns, preferably 5 to 20 microns, more preferably 12 to 17 microns.

In one embodiment of the invention, the fondant-based pharmaceutical composition comprises less than 0.5 percent of fats, or less than 0.1 percent of fats, or is totally free of fats.

In another embodiment of the invention, the fondant-based pharmaceutical composition is soft and deformable at room temperature. For example, the fondant-based pharmaceutical composition has a yield stress of 100 to about 100,000 Pascals. Preferably, the yield stress of the composition is in the range of about 1000 to about 80,000 Pascals, more preferably about 5000 to about 50,000 Pascals. Yield stress of the composition may be measured for example using the TA texture analyzer, model TA-XT2i, available from Texture Technologies Corp., Hamilton, MA, or the universal test systems available from Instron Corporation, Canton, MA. These instruments measure the force per unit area required to move or deform a material. Alternatively, the penetrometer method for measuring yield stress on materials of high consistency may be used, as set forth in Uhlherr, P.H.T., J. Guo, T.-N. Fang, C. Tiu, "Static measurement of yield stress using a cylindrical penetrometer," Korea-Australia Rheology: Journal, Vol. 14, No.1, March 2002 pp. 17-23.

In one embodiment of the invention, the fondant-based pharmaceutical composition comprises at least one hydrolase. The hydrolase is capable of hydrolyzing the carbohydrate contained within the composition upon activation by water. Suitable hydrolases include, but are not limited to glycosidases, such as invertase (sucrase), galactosidase, lactase (beta-galactosidase), maltase (alpha-galactosidase), xylase, and beta amylase, and mixtures thereof. Hydrolysis of the carbohydrate causes the fondant-based pharmaceutical composition to become softer and more viscous.

The amount of hydrolase present in the composition is that sufficient to hydrolyze at least a portion of the carbohydrate. The precise amount depends on both the nature of the carbohydrate and the nature of the hydrolase. In one embodiment of the invention wherein the composition comprises a hydrolase and the carbohydrate is sucrose, the hydrolase is invertase. Invertase is typically available as a liquid preparation in various strengths, e.g. single strength (2400 SU per ml), double strength (4000 SU per ml), and triple strength (10,000 SU per ml). One SU (Summer unit) is the amount of enzyme which produces 1 mg of invert sugar from 6 ml of a 5.4% sucrose solution at 20° C and pH 4.5 in 5 minutes. In one particular embodiment wherein the carbohydrate is sucrose and the hydrolase is invertase, the ratio of invertase to sucrose is typically from about 4,000 to about 13,000 SU of invertase per kilogram of sucrose.

The fondant-based pharmaceutical composition may be coated with one or more coatings to make a dosage form for administration of the active ingredient contained therein. In certain embodiments in which the fondant-based pharmaceutical composition is contained in a dosage form, the dosage form may comprise a core comprising the fondant-based pharmaceutical composition; and a first coating surrounding at least a portion of the core; and optionally an outer shell, surrounding at least a portion of the core and first coating.

In one embodiment of the invention, at least one coating comprises a water impermeable material. For example, the first coating may be substantially water impermeable, and may preferably comprise an insoluble edible material. In such embodiments, the first coating is particularly beneficial for protecting the fondant-based pharmaceutical composition in the core from moisture, enabling further coating without erosion of the core by the typically water-based coating solution.

In another embodiment, at least one coating is in the form of a hard shell. For example, one such hard shell may preferably comprise a crystallizable carbohydrate. Such carbohydrate based crunchy coatings are particularly beneficial for imparting a sweet taste, impact resistance, and elegant aesthetics to the dosage form, thus protecting the soft composition in the core. When a hard shell coating is employed it is preferred that an additional coating of water impermeable material underlie the hard shell.

In these embodiments the fondant-based core is preferably between about 12 and about 30 mm, e.g. from about 8 to about 20 mm, in length, width, diameter, or thickness.

Suitable insoluble edible materials for use in the coating include water-insoluble polymers, and low-melting hydrophobic materials. Preferred insoluble edible materials are selected from fats, waxes and chocolates. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof. Suitable low-melting hydrophobic materials include fats, fatty acid esters, phospholipids, and waxes. Examples of suitable fats include cocoa butter, hydrogenated vegetable oils such as for example hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable crystallizable carbohydrates for use in the coating include the monosaccharides and the oligosaccharides. Of the monosaccharides, the aldohexoses e.g., the D and L isomers of allose, altrose, glucose, mannose, gulose, idose, galactose, tagatose, talose, and the ketohexoses e.g., the D and L isomers of fructose and sorbose along with their hydrogenated analogs: e.g., glucitol (sorbitol), and mannitol are preferred. Of the oligosaccharides, the 1,2-disaccharides sucrose, trehalose, and turanose, the 1,4-disaccharides maltose, lactose, and cellobiose, and the 1,6-disaccharides gentiobiose and melibiose, as well as the trisaccharide raffmose are preferred along with the isomerized form of sucrose known as isomaltulose and its hydrogenated analog isomalt. Other hydrogenated forms of reducing disaccharides (such as maltose and lactose), for example, maltitol and lactitol are also preferred. Additionally, the hydrogenated forms of the aldopentoses: e.g., D and L ribose, arabinose, xylose, and lyxose and the hydrogenated forms of the aldotetroses: e.g., D and L erythrose and threose are preferred and are exemplified by xylitol and erythritol, respectively. Preferred crystallizable carbohydrates for use in hard shell coatings are preferably selected from the sugars and polyhedric alcohols. Preferred sugars include sucrose, dextrose, dextrose monohydrate, fructose, matlose, xylose, lactose, and mixtures thereof. Other suitable hard shell coatings include isomalt, cellulose derivatives, shellacs, and the like.

The fondant-based pharmaceutical composition of the present invention may be prepared using methods known in the confectionery arts. For example, fondant may be prepared by cooking a syrup consisting of sugar, corn syrup, and water in the appropriate ratio to a temperature of about 117° C to achieve a solids concentration of about 88%. Any commercial candy cooker may be used for this purpose such as is manufactured by APV Baker Perkins of the UK. Subsequent cooling and agitation of this concentrated syrup brings about a rapid crystallization of the sugar to yield a mass of very fine crystals (predominantly less than 20 microns) separated by thin films of a heavy syrup phase. Machines designed to cool and agitate the cooked sugar syrup are also available commercially from Otto Hansel of West Germany and APV Baker Perkins. In the APV Baker Perkins equipment, the cooked syrup is cooled by dropping it as a continuous stream onto a slowly rotating metal drum cooled internally by water. Once cooled to about 38° C, the supersaturated syrup is scraped from the drum and charged into a beater device. The beater device consists of a water cooled jacketed casing fitted inside with stationary pegs and rotating spindles that provide a high degree of agitation to the supersaturated syrup. The agitation induces nucleation in the syrup followed by a rapid crystallization of the sugar component into a mass of fine crystals. Temperature control by the water jacket removes the heat of crystallization and fondant flows from the beater at less than 43° C. Fondant machines of this kind may be operated in batch or continuous mode with outputs of about 500 kg per hour.

Alternately, the fondant-based pharmaceutical composition of the present invention may be prepared by mixing fondant grade sugar and water at room temperature.

The fondant based pharmaceutical composition can be advantageously made into a tablet, core, substrate, or the like (referred to below as a tablet) employing any process, for example compressing, molding, depositing, casting, or extruding. For example the fondant-based pharmaceutical composition may be deposited into a mold, cooled to a temperature at which the composition becomes solid, and removed from the mold as a core.

In certain embodiments of the invention, the fondant-based pharmaceutical composition of the invention may advantageously be made into a soft tablet by first compressing a relatively hard tablet containing a hydrolyzable carbohydrate and an effective amount of a hydrolase, which then becomes soft upon hydrolysis of the carbohydrate by the hydrolase in the presence of water.

In particular, a soft tablet may be made by a method comprising: (a) forming a tablet containing an active ingredient and a hydrolyzable carbohydrate to a hardness of 2 to 10 kp/cm2, preferably 4 to 10 kp/cm2; (b) adding water to the tablet before, during or after step (a); and (c) adding a hydrolase to the tablet before, during of after step (a).

In one embodiment of this method, the tablet is formed in step (a) by compression, for example using rotary compression or compacting roller technology such as a chilsonator or drop roller. Preferably, the tablet is made by compaction using a rotary tablet press. Preferably the compressed tablet has an initial hardness after compression of 2 to 10 kp/cm2, e.g. from 5 to 10 kp/cm2; and an initial friability after compression of less than 2%, e.g. less than 1%. These tablets, or cores, advantageously may be produced on conventional pharmaceutical equipment, and handled and further processed without breaking or chipping.

Before, during or after the tablet has been formed in step (a), water and a hydrolase are added to the active ingredient and the carbohydrate. For example, the water may be added by: a) applying water to the tablet surface after it has been formed, b) permitting water to be absorbed by the tablet during post-formation soft pan coating; c) exposing the tablet to a humid environment; d) adding water to the tablet via vacuum assistance; e) directly injecting water into the tablet; or combinations thereof.

Alternatively, water may be incorporated into the mix prior to tablet formation via high moisture granulation processing.

In an alternative embodiment, the hydrolase alone, or the hydrolase with water, may be added to the active ingredient and carbohydrate before or after tablet formation via any of the methods set forth above.

The amount of water used typically ranges from about 8 to about 15 weight percent of the tablet.

Upon contact of the carbohydrate, hydrolase, and water, the carbohydrate is hydrolyzed and the tablet softens. It is preferred that the tablet be allowed to stand for a period of time, preferably at least 24 hours, e.g. from 1 to 30 days, in order for softening to take place. Heat may optionally be applied to the tablet during this time. The hardness of the finished (cured) tablet is preferably in the range of 0 to 4 kp/cm², e.g. from 0.5 to 3.0 kp/cm².

In another embodiment of the invention, the tablet (with or without a hyrolase) is formed by molding, for example injection molding, thermal cycle molding as described in copending U.S. Application Serial No. 09/966,497 at pages 27-51, or thermal setting molding as described in copending U.S. Application Serial No. 09/966,450 at pages 57-63. Preferably thermal cycle molding or thermal setting molding is employed.

In the thermal setting molding method, the active ingredient, dispersed in a flowable material comprising the hydrolyzable carbohydrate and any other desired ingredients are injected in flowable form into a molding chamber. The flowable material may optionally comprise a solvent such as for example water, or organic solvents, or combinations thereof. The flowable material may optionally comprise up to about 10% of a thermal setting material as a processing aid. In embodiments in which a thermal setting material is employed, the flowable material is molded at a temperature sufficient for the thermal setting material to flow under an applied force but below the decomposition temperature of the active ingredient. The use of thermal setting materials may advantageously enable the fondant-based pharmaceutical composition to harden at a higher temperature. The flowable material is cooled and hardens in the molding chamber into a core (i.e., having the shape of the mold). In one embodiment the flowable material is substantially free of a thermal setting material. In a particularly preferred embodiment, the flowable material comprises or consists essentially of the active ingredient and confectionery fondant, which is a dispersion of carbohydrate crystals in a saturated carbohydrate solution.

According to this method, the starting material must be in flowable form. The starting material may be in the form of a suspension, or semi-solid paste. For example the flowable starting material may comprise solid carbohydrate crystals suspended in a saturated solution of carbohydrate in water.

Suitable thermal setting materials are any edible materials or mixtures of materials that are flowable at a temperature between 37 and 250°C, and harden or solidify at a temperature between -10 and 35°C. Preferred thermal setting materials include thermoplastic water swellable cellulose derivatives, thermoplastic water insoluble cellulose derivatrives, thermoplastic vinyl polymers, thermoplastic starches, thermplastic polyalkalene glycols, thermoplastic polyalkalene oxides, and amorphous sugar-glass, insoluble edible materials and the like, and derivatives, copolymers, and combinations thereof.

Examples of suitable thermoplastic water swellable cellulose derivatives include hydroxypropyl cellulose (HPC), hydroxypropylmethyl cellulose (HPMC), methyl cellulose (MC). Examples of suitable thermoplastic water insoluble cellulose derivatrives include cellulose acetate (CA), ethyl cellulose (EC), cellulose acetate butyrate (CAB), cellulose propionate. Examples of suitable thermoplastic vinyl polymers include polyvinyl alcohol (PVA) and polyvinyl pyrrolidone (PVP). Examples of suitable thermoplastic starches are disclosed for example in U.S. Patent No. 5,427,614. Examples of suitable thermoplastic polyalkalene glycols include polyethylene glycol. Examples of suitable thermoplastic polyalkalene oxides include polyethylene oxide having a molecular weight from 100,000 to 900,000 Daltons. Other suitable thermoplastic materials include sugar in the form on an amorphous glass such as that used to make hard candy forms.

Suitable insoluble edible materials for use as thermal setting materials include water-insoluble polymers, and low-melting hydrophobic materials. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof. Suitable low-melting hydrophobic materials include fats, fatty acid esters, phospholipids, and waxes. Examples of suitable fats include cocoa butter, hydrogenated vegetable oils such as for example hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

In the thermal cycle molding method, a thermal cycle molding module having the general configuration shown in Figure 3 of U.S. Application Serial No. 09/966,497 is employed. The thermal cycle molding module 200 comprises a rotor 202 around which a plurality of mold units 204 are disposed. The thermal cycle molding module includes a reservoir 206 (see Figure 4) for holding the fondant-based pharmaceutical composition to make the tablet. In addition, the thermal cycle molding module is provided with a temperature control system for rapidly heating and cooling the mold units. Figures 55 and 56 of the '497 application depict such a temperature control system 600.

In this embodiment, the mold units preferably comprise center mold assemblies 212 and upper mold assemblies 214 as shown in Figure 26C of the '497 application, which mate to form mold cavities having the desired shape of the tablet. As rotor 202 rotates, the opposing center and upper mold assemblies close. Fondant-based pharmaceutical composition, which is heated to a flowable state in reservoir 206, is injected into the resulting mold cavities. The temperature of the composition is then decreased, hardening the composition into tablets. The mold assemblies open and eject the tablets.

The tablet formed by molding in step (a) has a hardness in the range of about 2 to about 10, preferably about 2 to about 5, kp/cm². In this particular embodiment, the tablet need not be as robust. The particular apparatus and method used in this embodiment enables the processing of such soft friable materials without breaking.

Tablet hardness is used to describe the diametral breaking strength as measured by conventional pharmaceutical hardness testing equipment, such as a Schleuniger Hardness Tester. In order to compare values across different size tablets, or cores, the breaking strength must be normalized for the area of the break. This normalized value, expressed in kp/cm², is sometimes referred in the art as tablet tensile strength. A general discussion of tablet hardness testing is found in Leiberman et al., Pharmaceutical Dosage Forms - Tablets, Volume 2, 2nd ed., Marcel Dekker Inc., 1990, pp. 213 - 217, 327 - 329.

Tablets may be coated with one or more coatings by any suitable method, for example dipping, enrobing, spraying, ladeling, roller coating, or molding as known in the art. In one embodiment, for example, cooled tablets may be placed on a sheet of coating material, and enrobed with a first coating by pouring a melted flowable composition over the exposed surface of the tablet, and allowing the first coating composition to harden by cooling.

The so-coated tablets may, in turn, be further coated with an outer shell employing known methods, for example hard panning by spraying or ladeling a carbohydrate based solution onto the coated tablets in a conventional coating pan.

In one embodiment of the invention the coatings are applied by spraying in a coating pan, as known in the art.

In another embodiment of the invention the coatings are each applied by thermal cycle molding as described in copending U.S. Application Serial No. 09/966,497. In this embodiment, the coatings are applied using a thermal cycle molding module having the general configuration shown in Figure 3 therein. The thermal cycle molding module 200 comprises a rotor 202 around which a plurality of mold units 204 are disposed. The thermal cycle molding module includes reservoirs 206 (see Figure 4 therein) for holding flowable material used to make the coatings. In addition, the thermal cycle molding module is provided with a temperature control system for rapidly heating and cooling the mold units. Figures 55 and 56 of the '497 application depict the temperature control system 600.

The thermal cycle molding module is preferably of the type shown in Figure 28A of copending U.S. Application Serial No. 09/966,497, comprising a series of mold units 204. The mold units 204 in turn comprise upper mold assemblies 214, rotatable center mold assemblies 212 and lower mold assemblies 210 as shown in Figure 28C. Tablets comprising fondant-based pharmaceutical composition are continuously transferred to the mold assemblies, which then close over the tablets. The flowable material, which is heated to a flowable state in reservoir 206, is injected into the mold cavities created by the closed mold assemblies. The temperature of the flowable material is then decreased, hardening it. The mold assemblies open and eject the coated tablets. Coating is performed in two steps, each half of the tablets being coated separately as shown in the flow diagram of Figure 28B of the '497 application via rotation of the center mold assembly.

The coatings may comprise other components, such as natural or artificial sweeteners, colorants, flavors, plasticizers as known in the art.

In addition, the fondant-based pharmaceutical composition, the coating, or the overall dosage form may contain other conventional pharmaceutical additives, such as conventional dry binders like cellulose, cellulosic derivatives, polyvinyl pyrrolidone, starch, modified starch, and mixtures thereof, in particular microcrystalline cellulose; sweeteners like aspartame, acesulfame potassium, sucralose and saccharin; and lubricants, such as magnesium stearate, stearic acid, talc, and waxes, preservatives, flavors, antioxidants, surfactants, and coloring agents, and the like as known in the art.

The fondant-based pharmaceutical composition effectively taste masks and texture masks the active ingredient contained therein by providing the user with a silky smooth texture and little to no bitterness from the active ingredient. As a result, the composition is suitable for use in chewable or orally disintegrable dosage forms. In addition, tablets made from the composition may conveniently be consumed without water. Moreover, in contrast with known chewable dosage forms, the fondant-based pharmaceutical composition may accommodate relatively high doses of active ingredients, e.g. about 20 to about 50 weight percent while retaining a smooth, creamy mouthfeel.

Specific embodiments of the present invention are illustrated by way of the following examples. Unless otherwise stated, the percentages and ratios given below are by weight.

### Example 1

A batch of cores comprising fondant-based pharmaceutical composition according to the invention was made using the formulation set forth in Table 1 below:

**Table 1**

| **Ingredient** | | **Trade Name** | **Supplier** | **mg/ tab Theory** |
|---|---|---|---|---|
| Fondant [90% solids] | | | | |
| | Fondant Sugar | Amerfond | Domino | 355.01 |
| | Purified Water USP | NA | NA | 39.45 |
| Bob Syrup [Cooked to 87% solids] | | | | |
| | Sucrose NF | Extra Fine Granular | Domino | 989.85 |
| | Corn Syrup NF [42 DE/ 43] | | Roquette | 91.65 |
| | Purified Water USP | NA | NA | 140.54 |
| Coated Acetaminophen † | | NA | McNeil | 638.57 |
| Purified Water USP | | NA | NA | 81.75 |
| N&A Mint Flavor | | NA | Firmenich | 8.50 |
| Invertase | | Sucrovert Double Strength | Crompton & Knowels (CHR Hansen) | 3.19 |
| Sucralose | | Splenda | McNeil-PPC, Inc. | 1.49 |
| **TOTAL** | | | | 2,350.0 |

| | | | | |
|---|---|---|---|---|
| † Actual assay = 78.3% APAP | | | | |

Dry fondant sugar was placed in a planetary mixer bowl and slowly blended using a leaf blade until smooth and uniform as 10% w/w purified water was added. Invertase, Sucralose®, and flavor were added and the fondant mixture was uniformly blended. Bob syrup was prepared by cooking a mixture of granulated sucrose, 42 DE corn syrup, and purified water (approximately 75:7:18 % w/w) to 87% solids (approximately 115°C). The fondant mixture was then heated and maintained at 89-95°C. The Bob syrup was then added to the fondant mixture in the planetary mixer. Coated acetaminophen and purified water were added and the mixture was uniformly blended. While maintaining this mixture at 90-95°C, it was deposited into rubber molds.

As the warm, fluid, acetaminophen-containing, fondant-based pharmaceutical composition filled the mold cavities, the supersaturated sugar solution was shock crystallized and set as a firm solid mass containing suspended acetaminophen particles. Once set, the mold assemblies were opened and the molded cores were ejected from the mold.

### Example 2

A batch of cores as prepared in Example 1 were coated with a fat-containing coating to prepare dosage forms according to the invention as follows. Table 2 below sets forth the ingredients used:

**Table 2**

| **Ingredient** | **Trade Name** | **Supplier** | **mg/ tab Theory** |
|---|---|---|---|
| Fondant Centers, Example 1 | NA | NA | 2,350.0 |
| Partially Hydrogenated Vegetable Oil | CLSP870 | Loders Croaklan (Asher) | 250.0 |
| **TOTAL** | | | 2600.0 |

The cores according to Example 1 were first cooled in a conventional refrigerator to below room temperature. An excess of Partially Hydrogenated Vegetable Oil was melted with a stirring hot plate and maintained at 37-43°C. The cooled cores were placed in a Keith 16" conventional coating pan with 8 baffles. A Vortex Tube (model 3215) with the following settings 40 psi, 40C insert, exit temp 14-16°C was used to provide cool air to the tablet bed. The molten Partially Hydrogenated Vegetable Oil was applied to the moving tablet bed. During each application, enough Partially Hydrogenated Vegetable Oil was applied to completely wet the bed. The Partially Hydrogenated Vegetable Oil was allowed to completely solidify before the next application. Approximately 250 mg of Partially Hydrogenated Vegetable Oil per core was applied.

### Example 3

A hard sugar shell was applied to the fat-coated cores of Example 2 to prepare further dosage forms according to the invention using the coating formulation set forth in Table 3 below:

**Table 3**

| **Ingredient** | | **Trade Name** | **Supplier** | **Mg/ tab Theory** |
|---|---|---|---|---|
| Fat-coated Fondant Based Center, Example 2 | | NA | NA | 2600.00 |
| 67% Sucrose Solution | | | | |
| | Sucrose | Extra Fine Granular | Domino | 432.00 |
| | Purified Water USP | NA | NA | 212.78 |
| Corn Syrup NF [42 DE/ 43] | | | Roquette | 6.58 (5.26) |
| Opalux AS-11550 | | Opalux | Colorcon | 6.58 (3.68) |
| N&A Mint Flavor | | NA | Firmenich | 0.33 |
| Carnauba Wax NF [120 mesh powder] | | | Ross | 1.00 |
| **TOTAL** | | | | 3042.27 |

Sucrose was mixed with purified water at a ratio of 67:33. The mixture was heated to 60°C. After all of the sucrose was in solution, it was allowed to cool to less than 30°C. The final concentration was checked with a refractometer and adjusted to 67% solids. Colorant (Opalux® AS 11550), Flavor, and Corn Syrup 42 DE were added to the sucrose solution and mixed until uniform.

This coating solution was applied to the tumbling fat coated cores of Example 2 in a conventional coating pan in successive applications. Each application entailed an addition stage, a spreading stage, and a drying stage. In the addition stage, the solution was added to a bed of tumbling cores. Next, the solution was allowed to spread on the surface of the fat coated cores. Next, the drying stage employed blowing room temperature air over the bed to force the crystallization of the sucrose solution. The applications were repeated until the desired shell thickness was obtained. The sugar shell comprised approximately 25% of the final dosage form. Once the target weight was applied, the shell was polished in the coating pan with Carnauba Wax by applying the powder to the tumbling tablet bed.

### Example 4

Dosage forms according to the invention comprising acetaminophen were prepared on a commercial scale as follows. Table 5 lists the ingredients used.

**Table 5**

| **Ingredient** | | **Trade Name** | **Supplier** | **mg/ tab Theory** |
|---|---|---|---|---|
| Core | | | | |
| | Fondant Sugar | Amerfond | Domino | 1436.5 |
| | Coated Acetaminophen (90% Assay) | NA | McNeil | 555.0 |
| | Invertase | | Novo-Nordisk | 0.5 |
| | Magnesium Stearate NF | | Malinkrodt | 8.0 |
| Ice Plug | | | | |
| | Purified Water USP | NA | NA | 2.0 |
| Coating | | | | |
| | Partially Hydrogenated Vegetable Oil | CLSP870 | Loders Croaklan (Asher) | 250.0 |
| **TOTAL** | | | | 2300.0 |

Cores are prepared by the compression methods and apparatus described in copending U.S. Application Serial No. 09/966,509, pages 16-27, the disclosure of which is incorporated herein by reference. Specifically, the cores are made using a rotary compression module comprising a fill zone, insertion zone, compression zone, ejection zone, and purge zone in a single apparatus having a double row die construction as shown in Figure 6 of U.S. Application Serial No. 09/966,509. The dies of the compression module are preferably filled using the assistance of a vacuum, with filters located in or near each die. The purge zone of the compression module includes an optional powder recovery system to recover excess powder from the filters and return it to the dies.

The ingredients listed in Table 5 are first blended together to form a uniform powder mixture. The powder mixture is fed to the dies of the compression module. Ice plugs, made separately, are then inserted into powder mixture within each die. The powder mixture is compressed around the ice plugs, embedding the ice plugs within the cores.

The cores are received by a transfer device having the structure shown as 300 in Figure 3 of copending U.S. Application Serial No. 09/966,939. The transfer device comprises a plurality of transfer units 304 attached in cantilever fashion to a belt 312 as shown in Figures 68 and 69 of copending U.S. Application Serial No. 09/966,939. The transfer device rotates and operates in sync with the compression module and the thermal cycle molding module, described below, to which it is coupled. Transfer units 304 comprise retainers 330 for holding the cores as they travel around the transfer device.

The transfer device transfers the cores to a thermal cycle molding module, which applies the coating, partially hydrogenated vegetable oil, to the cores. The thermal cycle molding module is of the type shown in Figure 28A of copending U.S. Application Serial No. 09/966,939. The mold units 204 of the thermal cycle molding module comprise upper mold assemblies 214, rotatable center mold assemblies 212 and lower mold assemblies 210 as shown in Figure 28C. Cores are continuously transferred to the mold assemblies, which then close over the cores. Heated, flowable partially hydrogenated vegetable oil fills the mold assembliesm which are then rapidly cooled, hardening the oil into a coating. The mold assemblies open and eject the finished dosage forms. Coating is performed in two steps, each half of the cores being coated separately as shown in the flow diagram of Figure 28B of copending U.S. Application Serial No. 09/966,939 via rotation of the center mold assembly.

### Example 5

A taste test was performed to compare the dosage form of Example 3 with a conventional soft chewable dosage form containing the same level of acetaminophen active. A panel of 19, evaluating the fondant and dry chewable product, preferred the fondant based tablet overall by a margin of 15 to 4 and rated the attributes of mouthfeel, flavor, bitterness, and aftertaste as better than the conventional chewable form.

## Claims

1. A fondant-based pharmaceutical composition comprising an active ingredient and a carbohydrate, at least a portion of which carbohydrate is crystallized and has an average particle size of 2 to 35 microns, said composition having a moisture content in the range of 10 to 13 percent and wherein said active ingredient is in the form of particles having an average particle size of 200 to 1200 microns.

2. The composition of claim 1 wherein the composition comprises less than 0.5% of fats.

3. The composition of claim 1 further comprising at least one hydrolase in an amount sufficient to cause hydrolysis of at least a portion of the carbohydrate.

4. A dosage form comprising: a) a fondant-based pharmaceutical composition comprising an active ingredient and a carbohydrate, at least a portion of which carbohydrate is crystallized and has an average particle size of 2 to 35 microns, said composition having a moisture content in the range of 10 to 13 percent and said active ingredient being in the form of particles having an average particle size of 200 to 1200 microns; and b) at least one coating overlying said composition.

5. The dosage form of claim 4 further comprising at least one hydrolase in an amount sufficient to cause hydrolysis of at least a portion of the carbohydrate.

6. A method for making a soft tablet comprising:
(a) forming a tablet containing an active ingredient wherein said active ingredient is in the form of particles having an average particle size of 200 to 1200 microns, and a hydrolyzable carbohydrate to a hardness of 3 to 10 kp/cm²;
(b) adding water to the tablet before, during or after step (a); and
(c) adding a hydrolase to the tablet before, during or after step (a).

7. The method of claim 6 further comprising applying at least one coating to the tablet.

8. A dosage form comprising:
a) a core comprising the fondant based pharmaceutical composition of claim 1;
b) a first coating surrounding at least a portion of the core, wherein the first coating comprises an insoluble edible material; and
c) an outer shell surrounding at least a portion of the core and the first coating, wherein the outer shell comprises a crystallizable carbohydrate.

## Patentansprüche

1. Pharmazeutische Zusammensetzung auf Fondantbasis, umfassend einen aktiven Inhaltsstoff und ein Kohlehydrat, wobei wenigstens ein Teil des Kohlehydrats kristallisiert ist und eine durchschnittliche Teilchengröße von 2 bis 35 Mikrons besitzt, wobei die Zusammensetzung einen Feuchtegehalt im Bereich von 10 bis 13 Prozent besitzt und wobei der aktive Inhaltsstoff in Form von Teilchen mit einer durchschnittlichen Teilchengröße von 200 bis 1200 Mikrons vorliegt.

2. Zusammensetzung nach Anspruch 1, wobei die Zusammensetzung weniger als 0,5 % Fette umfasst.

3. Zusammensetzung nach Anspruch 1, die weiter wenigstens eine Hydrolase in einer Menge umfasst, die ausreichend ist, um Hydrolyse wenigstens eines Teils des Kohlehydrats zu bewirken.

4. Darreichungsform, umfassend: a) eine pharmazeutische Zusammensetzung auf Fondantbasis, umfassend einen aktiven Inhaltsstoff und ein Kohlehydrat, wobei wenigstens ein Teil des Kohlehydrats kristallisiert ist und eine durchschnittliche Teilchengröße von 2 bis 35 Mikrons besitzt, wobei die Zusammensetzung einen Feuchtegehalt im Bereich von 10 bis 13 Prozent besitzt und wobei der aktive Inhaltsstoff in Form von Teilchen mit einer durchschnittlichen Teilchengröße von 200 bis 1200 Mikrons vorliegt; und b) wenigstens einen Überzug, der über der Zusammensetzung liegt.

5. Darreichungsform nach Anspruch 4, die weiter wenigstens eine Hydrolase in einer Menge umfasst, die ausreichend ist, um Hydrolyse wenigstens eines Teils des Kohlehydrats zu bewirken.

6. Verfahren zur Herstellung einer weichen Tablette, umfassend:
a) Ausbilden einer Tablette, die einen aktiven Inhaltsstoff, wobei der aktive Inhaltsstoff in Form von Teilchen mit einer durchschnittlichen Teilchengröße von 200 bis 1200 Mikrons vorliegt, und ein hydrolysierbares Kohlehydrat enthält, zu einer Härte von 3 bis 10 kp/cm²;
b) Zugeben von Wasser zur Tablette vor, während oder nach Schritt (a); und
c) Zugeben einer Hydrolase zur Tablette vor, während oder nach Schritt (a).

7. Verfahren nach Anspruch 6, das weiter das Aufbringen wenigstens eines Überzugs auf die Tablette umfasst.

8. Darreichungsform, umfassend:
a) einen Kern, der die pharmazeutische Zusammensetzung auf Fondantbasis nach Anspruch 1 umfasst:
b) einen ersten Überzug, der wenigstens einen Teil des Kerns umgibt, wobei der erste Überzug ein unlösliches essbares Material umfasst; und
c) einen äußeren Mantel, der wenigstens einen Teil des Kerns und des ersten Überzugs umgibt, wobei der äußere Mantel ein kristallisierbares Kohlehydrat umfasst.

## Revendications

1. Composition pharmaceutique à base d'un fondant comprenant un principe actif et un glucide, une partie au moins dudit glucide étant cristallisée et ayant une taille de particule moyenne de 2 à 35 microns, ladite composition ayant une teneur d'humidité dans la plage de 10 à 13 % et dans laquelle ledit principe actif est sous la forme de particules ayant une taille de particule moyenne de 200 à 1 200 microns.

2. Composition selon la revendication 1, dans laquelle la composition comprend moins de 0,5 % de matières grasses.

3. Composition selon la revendication 1 comprenant, en outre, au moins une hydrolase en une quantité suffisante pour induire l'hydrolyse d'au moins une partie du glucide.

4. Forme pharmaceutique comprenant : a) une composition pharmaceutique à base d'un fondant comprenant un principe actif et un glucide, une partie au moins dudit glucide étant cristallisée et ayant une taille de particule moyenne de 2 à 35 microns, ladite composition ayant une teneur d'humidité dans la plage de 10 à 13 % et ledit principe actif étant sous la forme de particules ayant une taille de particule moyenne de 200 à 1 200 microns ; et b) au moins un enrobage enrobant ladite composition.

5. Forme pharmaceutique selon la revendication 4 comprenant, en outre, au moins une hydrolase en une quantité suffisante pour induire l'hydrolyse d'au moins une partie du glucide.

6. Procédé de fabrication d'un comprimé mou comprenant :
(a) la mise en forme d'un comprimé contenant un principe actif, ledit principe actif étant sous la forme de particules ayant une taille de particule moyenne de 200 à 1 200 microns et un glucide hydrolysable jusqu'à obtention d'une dureté de 3 à 10 kp/cm² ;
(b) l'ajout d'eau au comprimé avant, pendant ou après l'étape (a) ; et
(c) l'ajout d'une hydrolase au comprimé avant, pendant ou après l'étape (a).

7. Procédé selon la revendication 6 comprenant, en outre, l'application d'au moins un enrobage au comprimé.

8. Forme pharmaceutique comprenant :
a) un coeur comprenant la composition pharmaceutique à base d'un fondant selon la revendication 1 ;
b) un premier enrobage entourant au moins une partie du coeur, le premier enrobage comprenant une substance comestible insoluble ; et
c) une coque extérieure entourant au moins une partie du coeur et du premier enrobage, la coque extérieure comprenant un glucide cristallisable.
